# EUROPÄISCHE PATENTANMELDUNG

(11) **EP 1 914 218 A1**
(43) Veröffentlichungstag der Anmeldung: **23.04.2008**
(21) Anmeldenummer: 07112341.8
(22) Anmeldetag: 12.07.2007
(51) Int. Cl.: C07C 29/136, C07C 31/20

(54) **Verfahren zur Herstellung von 1,2-Diolen aus Carbonylverbindungen**

(30) Priorität: 07.09.2006 DE 102006041941
(71) Anmelder: Evonik Degussa GmbH, 40474 Düsseldorf (DE)
(72) Erfinder: Krimmer, Hans-Peter, Dr., 84558 Kirchweidach (DE); Sans, Jürgen, Dr., 83308 Trostberg (DE); Theis, Christoph, Dr., 53859 Niederkassel (DE); Thalhammer, Franz, Dr., 83308 Trostberg (DE)

(57) **Zusammenfassung**

Es wird ein Verfahren zur Herstellung von 1,2-Diolen aus Carbonylverbindungen beschrieben, welches dadurch gekennzeichnet ist, dass man
a) zunächst eine Carbonylverbindung der allgemeinen Formel (I), worin R¹ und R² für H, einen ggf. substituierten geradkettigen oder verzweigten C₁-C₁₈-Alkylrest, einen ggf. substituierten Phenyl- oder C₅-C₆-Cycloalkylrest stehen, mit Blausäure zum entsprechenden Cyanhydrin umsetzt, dann
b) dass aus dem Verfahrensschritt a) erhaltene Cyanhydrin einer sauren Hydrolyse unterwirft und abschließend
c) die aus Verfahrensschritt b) erhaltene 2-Hydroxycarbonsäure mit Hilfe eines Edelmetallkatalysators auf Basis von Ruthenium und Rhenium katalytisch hydriert.

Auf diese Weise lassen sich 1,2-Diole in guten Ausbeuten und in sehr hoher Reinheit auf technisch einfache Weise (d. h. ohne Spezialapparaturen) herstellen.

## Beschreibung

Die vorliegende Erfindung betrifft ein dreistufiges Verfahren zur Herstellung von 1,2-Diolen ausgehend von Carbonylverbindungen, wobei als Zwischenprodukte die entsprechenden Cyanhydrine und 2-Hydroxycarbonsäuren gebildet werden.

Die Umsetzung von Carbonylverbindungen wie Aldehyden und Ketonen mit Blausäure in Gegenwart von basischen Katalysatoren ist entsprechend dem Stand der Technik hinreichend bekannt (vgl. bspw. den Review "Formations of Cyanhydrins" in Science of Synthesis (2004) 19, 235 - 284).

Auch für die anschließende Hydrolyse der Nitrilgruppe des Cyanhydrins sind schon zahlreiche Verfahren beschrieben worden, wobei diese Hydrolyse im sauren oder alkalischen pH-Bereich durchgeführt werden kann.

Bei der sauren Hydrolyse entstehen hierbei 2-Hydroxycarbonsäuren, während bei der alkalischen Hydrolyse auch Aminosäure-Salze gebildet werden. Für die saure Hydrolyse sind entsprechend dem Stand der Technik verschiedene Mineralsäuren oder saure Ionenaustauscher geeignet. So wird bspw. die Herstellung von Hydroxycarbonsäure durch saure Hydrolyse aus dem entsprechenden Cyanhydrin in JP H05-155 816 A offenbart. Ein weiteres Beispiel für die saure Hydrolyse von Cyanhydrinen zu 2-Hydroxycarbonsäuren wird in der Patentschrift SU 1011630 A beschrieben.

Für die abschließende katalytische Hydrierung der 2-Hydroxycarbonsäuren stehen entsprechend dem Stand der Technik verschiedene Katalysatorsysteme zur Verfügung.

So wird gemäß der WO 01/16 063 die Hydrierung von 2-Hydroxycarbonsäure mit Kupferhaltigen Katalysatoren bei sehr milder Bedingungen und geringen Drucken empfohlen. Gegenstand der WO 2005/077 870 ist die Hydrierung von 2-Hydroxycarbonsäuren mit Hilfe von dotierten Edelmetallkatalysatoren. Des Weiteren ist aus der WO 2003/093 208 die Verwendung von Ruthenium-haltigen Katalysatoren für die Reduktion von Carbonsäuren bekannt. Ferner werden Katalysatorsysteme auf Basis von Ruthenium und Rhenium zur Hydrierung von 2-Hydroxycarbonsäuren in den internationalen Patentanmeldungen WO 99/38 824 und WO 99/38 613 offenbart.

Schließlich wird gemäß der Patentschrift DE 32 42 749 C1 ein Verfahren zur Herstellung von 1,2-Diolen beschrieben, wobei zunächst a) in Gegenwart eines Palladium- oder Platinkatalysators oder in Gegenwart von metallischem Nickel sowie einer organischen bzw. anorganischen Säure (wie z. B. Salzsäure) oder eines sauren Ionenaustauschers die Hydrierung eines Cyanhydrins bei einem Wasserstoffdruck von weniger als 10 bar so lange fortgeführt wird, bis pro Mol eingesetztem Cyanhydrin 1 Mol Wasserstoff aufgenommen ist und anschließend b) bei einem Wasserstoffdruck von 10 bis 150 bar die Hydrierung bis zur Beendigung der Wasserstoffaufnahme fortgeführt wird. Auf diese Weise lassen sich die entsprechenden Diole in guten Ausbeuten herstellen. Nachteilig bei diesem Verfahren ist jedoch die Tatsache, dass die Rückgewinnung und Reaktivierung des Katalysators in Gegenwart des sauren Ionenaustauschers technisch sehr aufwändig und kostenintensiv ist. Auch die Regenerierung des Ionenaustauschers in Gegenwart des Ruthenium/Rhenium-Katalysators gestaltet sich ebenfalls technisch schwierig.

Schließlich bringt das Arbeiten mit Salzsäure beim Verfahren gemäß DE 32 42 749 C1 erhebliche Werkstoffprobleme mit sich, da die Hydrierung in Gegenwart von Chlorid-lonen durchgeführt wird und deshalb diese Reaktionsstufe wegen der damit verbundenen Korrosionsproblemen nur in Autoklaven aus speziellen Werkstoffen erfolgen kann.

Der vorliegenden Erfindung lag daher die Aufgabe zugrunde, ein Verfahren zur Herstellung von 1,2-Diolen aus Carbonylverbindungen zu entwickeln, welches die genannten Nachteile des Standes der Technik nicht aufweist, sondern die Herstellung von 1,2-Diolen in guten Ausbeuten ermöglicht und gleichzeitig - im Hinblick auf die Durchführung und Aufarbeitung - auf technisch einfache Weise durchgeführt werden kann.

Diese Aufgabe wurde erfindungsgemäß dadurch gelöst, dass man a) zunächst eine Carbonylverbindung der allgemeinen Formel (I) 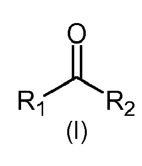 worin
R¹ und R² für H, einen ggf. substituierten geradkettigen oder verzweigten C₁-C₁₈-Alkylrest, einen ggf. substituierten Phenyl- oder C₅-C₆-Cycloalkylrest stehen, mit Blausäure zum entsprechenden Cyanhydrin umsetzt, dann b) das aus Verfahrensschritt a) erhaltene Cyanhydrin einer sauren Hydrolyse unterwirft und abschließend c) die aus Verfahrensschritt b) erhaltene 2-Hydroxycarbonsäure mit Hilfe eines Edelmetallkatalysators auf Basis von Ruthenium und Rhenium katalytisch hydriert. Es hat sich nämlich überraschenderweise gezeigt, dass man auf diese Weise 1,2-Diole in guten Ausbeuten und in technisch einfacher Weise (d. h. ohne Spezialapparaturen) herstellen kann. Außerdem weisen die erfindungsgemäß hergestellten 1,2-Diole eine sehr hohe Reinheit auf, was ebenfalls nicht vorhersehbar war.

Das Verfahren entsprechend der vorliegenden Erfindung besteht aus den drei Reaktionsstufen a), b) und c). In der ersten Reaktionsstufe a) wird eine Carbonylverbindung der allgemeinen Formel (I) 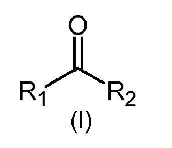 mit Blausäure zum entsprechenden Cyanhydrin umgesetzt. In der allgemeinen Formel (I) stehen R¹ und R² unabhängig voneinander für H, einen ggf. substituierten geradkettigen oder verzweigten C₁-C₁₈-Alkylrest sowie einen ggf. substituierten Phenyl- oder C₅-C₆-Cycloalkylrest. Im Falle von Alkyl- oder Cycloalkylresten kann die Carbonylverbindung noch mindestens einen Substituenten der Reihe OH, NH₂ oder OR³ aufweisen, wobei R³ einen C₁-C₈-Alkylrest bedeutet. Im Falle von Phenylresten kann die Carbonylverbindung der allgemeinen Formel (I) mindestens noch einen OH- oder NH₂-Substituenten aufweisen. Gemäß einer bevorzugten Ausführungsform werden als Carbonylverbindungen Aldehyde und insbesondere Butyraldehyd eingesetzt. Die Temperaturen in der Reaktionsstufe a) können in weiten Grenzen variiert werden, doch hat es sich aus Gründen der Wirtschaftlichkeit als besonders vorteilhaft erwiesen, diese Reaktion bei Temperaturen von 0 bis 100°C und insbesondere 0 bis 30°C durchzuführen. Besonders vorteilhaft ist die Durchführung der Reaktionsstufe a) ohne Lösemittel oder in Gegenwart eines Lösemittels ausgewählt aus der Gruppe Wasser, Alkohole, Ether oder Mischungen davon, wobei bei den Alkoholen C₁-C₄-Alkohole und bei den Ethern Diethylether und THF bevorzugt eingesetzt werden. Außerdem kann der Verfahrensschritt a) in Gegenwart eines basischen Katalysators durchgeführt werden, wobei vorzugsweise organische Amine wie z. B. Triethylamin herangezogen werden können. Vorzugsweise wird der basische Katalysator in einer Menge von 0,1 bis 10 Mol.-%, bezogen auf die Carbonylverbindung, eingesetzt. Gemäß einer bevorzugten Ausführungsform arbeitet man in der Reaktionsstufe a) mit einem 0,1 bis 10%-igen molaren Überschuss an Blausäure, bezogen auf die eingesetzte Carbonylverbindung. Nach Abschluss der Reaktionsstufe a) empfiehlt es sich, dass gebildete Cyanhydrin durch pH-Wert-Einstellung des Reaktionsgemisches auf 1,0 bis 6,0 mit Hilfe von Säuren wie z. B. Salzsäure zu stabilisieren.

In der nachfolgenden zweiten Reaktionsstufe b) wird das aus Verfahrensschritt a) erhaltene Cyanhydrin einer sauren Hydrolyse unterworfen. Die saure Hydrolyse wird vorzugsweise mit Hilfe einer Mineralsäure, insbesondere in Form einer wässrigen Lösung, oder in Gegenwart eines sauren Ionenaustauschers durchgeführt. Als Mineralsäure hat sich hierbei insbesondere eine 20 bis 37%-ige Salzsäure oder eine 50 bis 80%-ige Schwefelsäure bewährt. Das Verhältnis von Mineralsäure zu Cyanhydrin ist relativ unkritisch, doch hat es sich als besonders vorteilhaft erwiesen, die Mineralsäure in einem Säureequivalent-Verhältnis zum Cyanhydrin von 1,0 bis 10,0:1 und besonders bevorzugt von 1,2 bis 2,0:1 einzusetzen. Der Hydrolyseschritt b) wird vorzugsweise bei erhöhten Temperaturen von 30 bis 130°C und insbesondere von 60 bis 110°C durchgeführt. Es ist im Rahmen der vorliegenden Erfindung ohne weiteres möglich, die Hydrolyse unter erhöhten Drücken durchzuführen, wobei Siedebedingungen unter Normaldruck bevorzugt werden. Nach Abschluss der sauren Hydrolyse wird gemäß einer bevorzugten Ausführungsform der pH-Wert des Reaktionsgemisches mit wässriger Natronlauge auf einen pH-Wert von 0 bis 4 eingestellt, das Lösemittel abgetrennt und die 2-Hydroxycarbonsäure durch Kristallisation oder Extraktion mit einem organischen Lösemittel gewonnen. Im Falle der Extraktion wird die 2-Hydroxycarbonsäure vorzugsweise anschließend durch Destillation unter vermindertem Druck bei 0,1 bis 100 mbar aufgereinigt.

In der dritten Reaktionsstufe c) wird die aus Verfahrensschritt b) erhaltene 2-Hydroxycarbonsäure mit Hilfe eines Edelmetallkatalysators auf Basis von Ruthenium und Rhenium katalytisch hydriert. Der Edelmetallkatalysator in Stufe c) weist hierbei einen bevorzugten Edelmetallgehalt von 1 bis 10 Gew.-% auf, wobei als Trägermaterial vorzugsweise Aktivkohle eingesetzt wird. Außerdem besitzt der im Hydrierschritt c) eingesetzte Ruthenium/Rhenium-Katalysator einen bevorzugten Gehalt an Ruthenium und Rhenium von jeweils 1 bis 10 Gew.-%. Die Reaktionsbedingungen im Hydrierschritt c) können ebenfalls in weiten Grenzen variiert werden. Als besonders vorteilhaft haben sich Temperaturen von 100 bis 300°C und besonders bevorzugt von 180 bis 200°C sowie Wasserstoffdrücke von 50 bis 300 bar und als besonders bevorzugt von 200 bis 250 bar erwiesen. Gemäß einer bevorzugen Ausführungsform kann die Hydrierstufe c) auch in zwei Schritten vorgenommen werden. Die Menge des eingesetzten Katalysators beträgt in der Regel von 0,1 bis 7,5 Gew.-% und insbesondere von 2 bis 5 Gew.-%, jeweils bezogen auf die Menge an eingesetzter 2-Hydroxycarbonsäure. Die Hydrierung gemäß Reaktionsstufe c) ist je nach Reaktionsbedingungen nach spätestens 20 Stunden vorzugsweise nach 1 bis 12 Stunden und besonders bevorzugt nach 7 bis 10 Stunden beendet. Nach Abschluss der Reaktionsstufe c) wird der Ruthenium/Rhenium-Katalysator durch Filtration vom Rohprodukt abgetrennt und kann gemäß einer bevorzugten Ausführungsform recyclisiert werden. Das erhaltene Reaktionsprodukt, bestehend aus dem entsprechenden 1,2-Diol kann bspw. durch Destillation isoliert bzw. aufgereinigt werden. Beim Verfahren entsprechend der vorliegenden Erfindung wird das 1,2-Diol in guten Ausbeuten von > 60% und sehr hohen Reinheiten von > 98% erhalten. Vorzugsweise wird in dem erfindungsgemäßen Verfahren 1,2-Pentadiol erhalten.

Die nachfolgenden Beispiele sollen die vorliegende Erfindung näher veranschaulichen.

### Beispiele

### Beispiel 1:

In einem Glaskolben werden 72 g (1,0 mol) Butyraldehyd bei Raumtemperatur vorgelegt und mit 0,5 g Triethylamin als Katalysator gemischt. Dann werden 27,5 g (1,02 mol) Blausäure temperaturgeregelt bei 15 - 20 °C zudosiert und etwa 1 Stunde bei Raumtemperatur nachgerührt. Mit Salzsäure wird ein pH-Wert von 2 - 4 eingestellt, um das gebildete Cyanhydrin zu stabilisieren.

In einem zweiten Kolben werden 243 g (2,0 mol) Salzsäure 30 %ig bei 50 °C vorgelegt und das Cyanhydrin aus Kolben 1 innerhalb von 30 Minuten zudosiert. Während der Zugabe wird die Temperatur bis zum Rückfluss (bei 106 °C) erhöht. Im Anschluss wird noch 1 Stunde unter Rückfluss gerührt. Der Säureüberschuss wird dann mit wässriger Natronlauge 40 %ig bis pH 2 neutralisiert.

Das erhaltene Reaktionsgemisch wird mit 200 ml Methyl-tert.-butylether versetzt und die wässrige Phase abgetrennt. In der organischen Phase befindet sich 2-Hydroxy-pentansäure in einer Ausbeute > 90 %, die nach Abdestillieren des Lösemittels unter Vakuum von 1 mbar bei 140 - 150 °C destilliert wird. Die Ausbeute beträgt 73,8 g (71 % d.Th.).

29,5 g (0,25 mol) der so erhaltenen 2-Hydroxy-pentansäure werden in 470,5 g Wasser gelöst und diese Mischung nach Zugabe von 5,0 g wasserfeuchtem Katalysator auf Aktivkohle (*Belegung:* 3 % Ru und 2 % Re) in einem 1 L-Autoklaven bei einem Wasserstoffdruck von 250 bar und bei einer Reaktionstemperatur von 190°C innerhalb von 8 Stunden erschöpfend hydriert.

Nach Reaktionsende und Kühlung wird der Katalysator abfiltriert und das Filtrat unter Verwendung eines Rotationsverdampfers mit Kolonnenaufsatz bei vermindertem Druck destillativ vom Wasser befreit.

Der resultierende Rückstand wird dann, unter Verwendung einer Kurzweg-DestillationsApparatur mit kurzer Kolonne, einer fraktionierenden Hochvakuum-Destillation unterworfen.

Hierbei werden 16,8 g 1,2-Pentandiol mit einer GC-Reinheit (nach Silylieren) von 98,7 % erhalten, das entspricht einer Ausbeute von 64,6 % d.Th. bezogen auf die eingesetzte 2-Hydroxy-pentansäure.

### Beispiel 2:

In einem Glaskolben werden 360 g (5,0 mol) Butyraldehyd und 300 ml Wasser bei Raumtemperatur vorgelegt und mit 2,5 g Triethylamin als Katalysator gemischt. Dann werden 137 g (5,1 mol) Blausäure temperaturgeregelt bei 15 - 20 °C zudosiert und etwa 1 Stunde bei Raumtemperatur nachgerührt. Mit Schwefelsäure 80 %ig wird ein pH-Wert von 2 - 4 eingestellt, um das gebildete Cyanhydrin zu stabilisieren.

In einem zweiten Kolben werden 980 g (8,0 mol) Schwefelsäure 80 %ig bei 90 °C vorgelegt und das Cyanhydrin aus Kolben 1 innerhalb von 60 Minuten zudosiert. Während der Zugabe wird die Temperatur auf 105 - 110 °C erhöht. Im Anschluss wird noch 3 Stunden bei dieser Temperatur gerührt. Der Säureüberschuss wird nach dem Abkühlen mit wässriger Natronlauge 40 %ig bis pH 2 neutralisiert.

Das erhaltene Reaktionsgemisch wird mit 1000 ml Methyl-tert.-butylether versetzt und die wässrige Phase abgetrennt. In der organischen Phase befindet sich 2-Hydroxy-pentansäure zusammen mit ca. 10 % nicht hydrolisiertem Butyraldehyd-cyanhydrin.

Nach Abdestillieren des Lösemittels wird das Produkt zusammen mit dem Cyanhydrin unter Vakuum von 1 mbar bei 140 - 150 °C destilliert. Die Ausbeute beträgt dabei 338,0 g (51,5 % d.Th. berechnet auf eine Reinheit von 90 %).

In einen 1 L-Autoklaven wird eine Suspension bestehend aus 400 g Wasser und 5,0 g wasserfeuchtem Katalysator (*Belegung:* 8 % Ru und 1 % Re) eingefüllt und bei 190 °C und 250 bar Wasserstoff für die Dauer von 2 Stunden vorhydriert.

Anschließend werden, nach Abkühlung und Entspannung, zur Katalysator-Suspension 156,0 g 2-Hydroxy-pentansäure - die noch einen Anteil von ca. 10 % an Butyraldehyd-cyanhydrin enthält - zugesetzt und nachfolgend das Reaktionsgemisch bei einer Reaktionstemperatur von 190 °C und einem Wasserstoffdruck von 250 bar im Verlauf von 14 Stunden bis zur Beendigung der Wasserstoffaufnahme hydriert.

Nach Katalysator-Abtrennung wird im Filtrat durch acidimetrische Titration der Umsatz der Hydrierung mit 96 % ermittelt, die weitere destillative Aufarbeitung des Filtrates erfolgt analog Beispiel 1.

Hierbei werden 89,2 g 1,2-Pentandiol mit einer Reinheit von 98,1 % erhalten; dies entspricht - bezogen auf eingesetzte 2-Hydroxy-pentansäure - einer Ausbeute von 72,1 % d.Th.

### Beispiel 3:

In einem Glaskolben werden 72 g (1,0 mol) Butyraldehyd bei Raumtemperatur vorgelegt und mit 0,5 g Triethylamin als Katalysator gemischt. Dann werden 27,5 g (1,02 mol) Blausäure temperaturgeregelt bei 15 - 20 °C zudosiert und etwa 1 Stunde bei Raumtemperatur nachgerührt. Mit Salzsäure wird ein pH-Wert von 2 - 4 eingestellt, um das gebildete Cyanhydrin zu stabilisieren.

In einem zweiten Kolben werden 243 g (2,0 mol) Salzsäure 30 %ig bei 50 °C vorgelegt und das Cyanhydrin aus Kolben 1 innerhalb von 30 Minuten zudosiert. Während der Zugabe wird die Temperatur bis zum Rückfluss (bei 106 °C) erhöht. Im Anschluss wird noch 1 Stunde unter Rückfluss gerührt. Der Säureüberschuss wird dann mit wässriger Natronlauge 40 %ig bis pH 2 neutralisiert.

Das erhaltene Reaktionsgemisch wird mit 200 ml Methyl-tert.-butylether versetzt und die wässrige Phase abgetrennt. Die organische Phase wird mit 200 ml Wasser ausgeschüttelt und von der Wasserphase abgetrennt. Sie enthält 2-Hydroxy-pentansäure in einer Ausbeute > 90 %. Das organische Lösemittel wird destillativ abgetrennt und der Rückstand der Hydrierung (Stufe c) unterworfen.

Dazu wird in einem 1 L-Autoklaven eine Suspension bestehend aus 400 g Wasser und 5,0 g wasserfeuchtem Katalysator (*Belegung:* 8 % Ru und 1 % Re) eingefüllt und bei 190 °C und 250 bar Wasserstoff für die Dauer von 2 Stunden vorhydriert.

Anschließend wird, nach Abkühlung und Entspannung, zur Katalysator-Suspension die rohe 2-Hydroxy-pentansäure zugesetzt und nachfolgend das Reaktionsgemisch bei einer Reaktionstemperatur von 190 °C und einem Wasserstoffdruck von 250 bar im Verlauf von 14 Stunden bis zur Beendigung der Wasserstoffaufnahme hydriert.

Nach Katalysator-Abtrennung wird im Filtrat durch acidimetrische Titration der Umsatz der Hydrierung mit 96 % ermittelt, die weitere destillative Aufarbeitung des Filtrates erfolgt analog Beispiel 1.

Hierbei werden 68,6 g 1,2-Pentandiol mit einer Reinheit von 98,1 % erhalten; dies entsprichtbezogen auf eingesetzten Butyraldehyd - einer Ausbeute von 66,0 % d.Th.

## Patentansprüche

1. Verfahren zur Herstellung von 1,2-Diolen aus Carbonylverbindungen,
**dadurch gekennzeichnet,**
**dass** man
a) zunächst eine Carbonylverbindung der allgemeinen Formel (I) 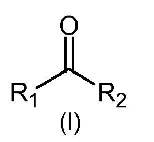 worin
R¹ und R² für H, einen ggf. substituierten geradkettigen oder verzweigten C₁C₁₈-Alkylrest, einen ggf. substituierten Phenyl- oder C₅-C₆-Cycloalkylrest stehen,
mit Blausäure zum entsprechenden Cyanhydrin umsetzt,
dann
b) das aus Verfahrensschritt a) erhaltene Cyanhydrin einer sauren Hydrolyse unterwirft und abschließend
c) die aus Verfahrensschritt b) erhaltene 2-Hydroxycarbonsäure mit Hilfe eines Edelmetall-Katalysators auf Basis von Ruthenium und Rhenium katalytisch hydriert.

2. Verfahren nach Anspruch 1,
**dadurch gekennzeichnet,**
**dass** R¹ und R² im Falle von Alkyl- oder Cycloalkyl-Resten mindestens einen Substituenten der Reihe OH, NH₂ oder OR³ (mit R³ = C₁-C₈-Alkyl) und im Falle von Phenylresten mindestens einen OH- oder NH₂-Substituenten aufweisen.

3. Verfahren nach Anspruch 1 oder 2,
**dadurch gekennzeichnet,**
**dass** man die Reaktionsstufe a) bei Temperaturen von 0 bis 100 °C, insbesondere 0 bis 30 °C, durchführt.

4. Verfahren nach einem der Ansprüche 1 bis 3,
**dadurch gekennzeichnet,**
**dass** die Stufe a) in Gegenwart eines Lösemittels, ausgewählt aus der Gruppe Wasser, Alkohole, Ether oder Mischungen davon, erfolgt.

5. Verfahren nach einem der Ansprüche 1 bis 4,
**dadurch gekennzeichnet,**
**dass** man in der Stufe a) die Blausäure in einem 5 bis 10 %igen molaren Überschuss bezogen auf die eingesetzte Carbonylverbindung einsetzt.

6. Verfahren nach einem der Ansprüche 1 bis 5,
**dadurch gekennzeichnet,**
**dass** der Verfahrensschritt a) in Gegenwart eines basischen Katalysators und vorzugsweise in Gegenwart eines organischen Amins, wie z. B. Triethylamin, durchgeführt wird.

7. Verfahren nach einem der Ansprüche 1 bis 6,
**dadurch gekennzeichnet,**
**dass** man den basischen Katalysator in einer Menge von 0,1 bis 10 Mol-%, bezogen auf die eingesetzte Carbonylverbindung, einsetzt.

8. Verfahren nach einem der Ansprüche 1 bis 7,
**dadurch gekennzeichnet,**
**dass** man nach Abschluss der Reaktionsstufe a) das gebildete Cyanhydrin durch pH-Wert-Einstellung des Reaktionsgemisches auf 1,0 bis 6,0 stabilisiert.

9. Verfahren nach einem der Ansprüche 1 bis 8,
**dadurch gekennzeichnet,**
**dass** die saure Hydrolyse im Verfahrensschritt b) mit Hilfe einer Mineralsäure, insbesondere in Form einer wässrigen Lösung und besonders bevorzugt mit Hilfe einer 20 bis 37 %igen Salzsäure oder einer 50 bis 80 %igen Schwefelsäure und/oder in Gegenwart eines sauren Ionentauschers, durchgeführt wird.

10. Verfahren nach einem der Ansprüche 1 bis 9,
**dadurch gekennzeichnet,**
**dass** die Mineralsäure in einem Säureequivalent-Verhältnis zum Cyanhydrin von 1,0 bis 10,0 : 1 und vorzugsweise von 1,2 bis 2,0 : 1 eingesetzt wird.

11. Verfahren nach einem der Ansprüche 1 bis 10,
**dadurch gekennzeichnet,**
**dass** der Hydrolyseschritt b) bei erhöhten Temperaturen von 30 bis 130 °C und insbesondere von 60 bis 100 °C und ggf. unter erhöhten Drücken durchgeführt wird, wobei Siedebedingungen unter Normaldruck bevorzugt werden.

12. Verfahren nach einem der Ansprüche 1 bis 11,
**dadurch gekennzeichnet,**
**dass** vor dem Hydrierungsschritt c) die 2-Hydroxycarbonsäure durch Kristallisation oder Extraktion mit einem organischen Lösemittel ggf. nach pH-Wert-Erhöhung mit wässriger Natronlauge auf einen pH-Wert von 0 bis 4 sowie Abtrennung des Lösemittels aufgereinigt wird.

13. Verfahren nach einem der Ansprüche 1 bis 12,
**dadurch gekennzeichnet,**
**dass** man die 2-Hydroxycarbonsäure durch Destillation unter vermindertem Druck bei 0,1 bis 100 mbar aufreinigt.

14. Verfahren nach einem der Ansprüche 1 bis 13,
**dadurch gekennzeichnet,**
**dass** der Ruthenium/Rhenium-Katalysator im Hydrierschritt c) einen Edelmetallgehalt von 1 bis 10 Gew.-% aufweist.

15. Verfahren nach einem der Ansprüche 1 bis 14,
**dadurch gekennzeichnet,**
**dass** der Edelmetall-Katalysator in Stufe c) einen Gehalt an Ruthenium von 1 bis 10 Gew.-% und an Rhenium von 1 bis 10 Gew.-% besitzt.

16. Verfahren nach einem der Ansprüche 1 bis 15,
**dadurch gekennzeichnet,**
**dass** das Trägermaterial des Ruthenium/Rhenium-Katalysators aus Aktivkohle besteht.

17. Verfahren nach einem der Ansprüche 1 bis 16,
**dadurch gekennzeichnet,**
**dass** man die Hydrierung bei Temperaturen von 100 bis 300 °C und vorzugsweise von 180 bis 200 °C durchführt.

18. Verfahren nach einem der Ansprüche 1 bis 17,
**dadurch gekennzeichnet,**
**dass** der Hydrierungsschritt c) bei Wasserstoffdrücken von 50 bis 300 bar und vorzugsweise bei 200 bis 250 bar erfolgt.

19. Verfahren nach einem der Ansprüche 1 bis 18,
**dadurch gekennzeichnet,**
**dass** die Hydrierung über einen Zeitraum von maximal 20 Stunden, vorzugsweise innerhalb von 1 bis 12 Stunden und besonders bevorzugt innerhalb von 7 bis 10 Stunden durchgeführt wird.

20. Verfahren nach einem der Ansprüche 1 bis 19,
**dadurch gekennzeichnet,**
**dass** die eingesetzte Katalysatormenge von 0,1 bis 7,5 Gew.-% und insbesondere von 2 bis 5 Gew.-%, jeweils bezogen auf die Menge an 2-Hydroxycarbonsäure, beträgt.

21. Verfahren nach einem der Ansprüche 1 bis 20,
**dadurch gekennzeichnet,**
**dass** der Hydrierungskatalysator recyclisiert wird.

22. Verfahren nach einem der Ansprüche 1 bis 21,
**dadurch gekennzeichnet,**
**dass** das erhaltene 1,2-Diol durch Destillation isoliert und/oder gereinigt wird.

23. Verfahren nach einem der Ansprüche 1 bis 22,
**dadurch gekennzeichnet,**
**dass** als Diol das 1,2-Pentandiol erhalten wird.
